(19) Europäisches Patentamt European Patent Office Office européen des brevets

(11) **EP 4 243 024 A1**

(12) **EUROPEAN PATENT APPLICATION**
published in accordance with Art. 153(4) EPC

(43) Date of publication:
**13.09.2023 Bulletin 2023/37**

(21) Application number: **21889581.1**

(22) Date of filing: **04.11.2021**

(51) International Patent Classification (IPC):
*G16B 40/10* (2019.01)    *G16B 5/00* (2019.01)
*G16B 20/20* (2019.01)    *G16B 30/10* (2019.01)
*G16H 50/50* (2018.01)    *G16H 50/70* (2018.01)

(52) Cooperative Patent Classification (CPC):
**G16B 5/00; G16B 20/20; G16B 30/10; G16B 40/10;
G16H 50/50; G16H 50/70**

(86) International application number:
**PCT/KR2021/015884**

(87) International publication number:
**WO 2022/098116 (12.05.2022 Gazette 2022/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.11.2020 KR 20200145749**

(71) Applicant: **Samsung Life Public Welfare
Foundation
Seoul 04348 (KR)**

(72) Inventors:
• **KIM, Hee Jin
Seoul 06354 (KR)**
• **WON, Hong Hee
Seoul 06351 (KR)**
• **KIM, Hang Rai
Sejong 30153 (KR)**
• **SEO, Sang Won
Seoul 06647 (KR)**
• **NA, Duk Lyul
Seoul 06317 (KR)**
• **JUNG, Sang Hyuk
Siheung-si Gyeonggi-do 14995 (KR)**

(74) Representative: **Weickmann & Weickmann
PartmbB
Postfach 860 820
81635 München (DE)**

(54) **METHOD FOR PREDICTING POSSIBILITY OF BRAIN AMYLOID BETA ACCUMULATION**

(57)    The present invention relates to a method of predicting the likelihood of brain amyloid-beta deposition in order to predict the occurrence of diseases caused by brain amyloid-beta deposition, including Alzheimer's disease, in Koreans, and, more specifically, to a method of providing information for predicting the likelihood of brain amyloid-beta deposition, and a composition and a kit for predicting the likelihood of brain amyloid-beta deposition.

The method of providing information for predicting the likelihood of brain amyloid-beta deposition may effectively screen individuals with a high likelihood of brain amyloid-beta deposition at an early stage, thereby providing an appropriate method of inhibiting the development of a disease caused by brain amyloid-beta deposition or treating an individual having the disease.

EP 4 243 024 A1

**EP 4 243 024 A1**

Fig. 5

| Models | Discovery dataset (10-fold CV) | | Replication dataset |
|---|---|---|---|
| | AUC | 95% CI | AUC |
| Model 1: CF (sex, age, education) | 0.506 | 0.500-0.512 | 0.509 |
| Model 2: CF + *APOE ε4* | 0.723 | 0.717-0.729 | 0.693 |
| Model 3: CF + *APOE ε4* + rs6978259 | 0.748 | 0.742-0.755 | 0.713 |

2

## Description

### Technical Field

The present invention relates to a method of predicting the likelihood of brain amyloid-beta deposition in order to predict the onset of diseases caused by brain amyloid-beta deposition, including Alzheimer's disease, in Koreans, and more specifically, to a method of providing information predicting the likelihood of brain amyloid-beta deposition, and a composition and a kit for predicting the likelihood of brain amyloid-beta deposition.

### Background Art

Alzheimer's disease (AD) is a representative degenerative brain disease that accounts for about 70% of dementia, and is the most common cause of dementia in the elderly. Genetic factors play an important role in the pathogenesis of Alzheimer's disease (AD) because heritability is estimated to be 58% to 79%, which indicates a genetic predisposition. Apolipoprotein E (APOE) ε4 is a well-known risk variant for AD, and recent genome-wide association studies (GWAS) have discovered a number of genetic risk variants for AD. However, a large proportion of AD heritability is still unexplained.

Meanwhile, early changes in the brains of AD patients are deposition of amyloid-beta (Aβ) in the brain, followed by tau deposition, neurodegeneration, and cognitive impairment. That is, amyloid-beta begins to accumulate in the brain 10 to 15 years before clinical symptoms of AD appear. Therefore, detecting or discovering individuals with brain Aβ deposition is of utmost importance for the early diagnosis and early treatment of AD. Conventionally, deposition of amyloid-beta in the brain was examined by positron emission tomography (PET) or cerebrospinal fluid analysis. However, PET is mainly performed in large hospitals due to high imaging costs and limited access, and causes risk factors such as radiation exposure, and cerebrospinal fluid analysis has disadvantages in that an invasive lumbar puncture is required and reliability varies depending on organs. Therefore, there is a need for biomarkers capable of predicting amyloid-beta deposition in the brain in a low-cost and non-invasive manner.

Since genomes differ greatly between races, genetic factors associated with Alzheimer's disease and amyloid-beta deposition in the brain differ between races. Most of previous studies on Alzheimer's disease genes have been conducted on Europeans, and genetic variants studies on non-Europeans are insufficient. In addition, studies on brain amyloid-beta-related genes, early diagnostic markers for Alzheimer's disease, are very rare even in foreign countries (Yan Q, et al. Mol Psychiatry. 2018:1-13; Apostoloval LG et al. JAMA Neurol. 2018: 328-341; Raghavan NS, et al. JAMA Neurol. 2020:1288-1298) and have not been conducted in Korea.

Therefore, in order to predict brain amyloid-beta deposition, which is an early diagnostic marker for Alzheimer's disease in Koreans, by a low-cost and minimally invasive method (basic clinical information and genetic information), a predictive model based on Korean data is required. In addition, verification procedures should be performed to ensure that the same model works even in an independent cohort.

[Prior Art Documents]

[Patent Documents]

Korean Patent No. 10-2130929
Korean Patent No. 10-1989695
Korean Patent Application Publication No. 10-2020-0073156

### DISCLOSURE

### Technical Problem

An object of the present invention is to provide a method of providing information for predicting the likelihood of brain amyloid-beta deposition based on a combination of individual characteristics and genetic information.

Another object of the present invention is to provide a composition for predicting the likelihood of brain amyloid-beta deposition, which may be used to identify genetic information.

Still another object of the present invention is to provide a kit for predicting the likelihood of brain amyloid-beta deposition, the kit comprising the composition.

**Technical Solution**

The present inventors have conducted GWAS analysis to identify single nucleotide polymorphisms (SNPs) associated with brain amyloid-beta deposition using a large sample of the Korean population. As a result, the present inventors have identified six novel SNPs for amyloid-beta deposition and demonstrated their associations in an independent cohort of the Korean population. In addition, the present inventors have developed a novel amyloid-beta prediction model by combining these novel SNP genotypes with individual characteristics and APOE genotypes, thereby completing the present invention that provides a method of predicting the likelihood of brain amyloid-beta deposition in the Korean population and a related composition

One aspect of the present invention provides a method of providing information for predicting the likelihood of brain amyloid-beta deposition through a combination of demographic characteristics, APOE $\varepsilon4$ genotypes and SNP genotypes. More specifically, the method of providing information for predicting the likelihood of brain amyloid-beta deposition comprises steps of: a) collecting individual characteristics including sex, age, and education level from an individual; b) measuring an APOE $\varepsilon4$ genotype and at least one SNP genotype selected from the group consisting of rs73375428, rs6978259, rs2903923, rs3828947, rs6958464, and rs11983537, in a biological sample from the individual; and c) predicting the likelihood of brain amyloid-beta deposition by combining the collected individual characteristics and the measured APOE $\varepsilon4$ genotype and SNP genotype.

As used herein, "likelihood of brain amyloid-beta deposition" means the risk of brain amyloid-beta deposition in an individual, and it can be said that the higher the risk, the higher the likelihood of developing a disease caused by amyloid-beta deposition. Here, the disease caused by amyloid-beta deposition may be Alzheimer's disease, Lewy body dementia, cerebral amyloid angiopathy, etc., without being limited thereto. This prediction of the likelihood of brain amyloid-beta deposition is distinct from techniques for diagnosing Alzheimer's disease or Alzheimer's dementia in an individual. In the present invention, a case where amyloid-beta is likely to be deposited in the brain or has already been deposited in the brain may be determined as positive.

Steps a) to c) will now be described in detail

Step a) is a process of collecting individual characteristics from an individual that needs to be tested for brain amyloid-beta deposition.

The individual is a subject in whom the likelihood of amyloid-beta deposition in the cerebrum is to be predicted, and may be a patient having a family history of a disease caused by brain amyloid-beta deposition (e.g., Alzheimer's disease, Lewy body dementia, or cerebral amyloid angiopathy) or suspected of having a brain disease due to some clinical symptoms due to memory loss, language impairment, and judgment decline, or may be a person with no specific clinical symptoms.

The individual characteristics are basic characteristics of an individual, such as demographic characteristics, and may include sex, age, and education level.

The sex refers to a biological sex, and it is known that women are more likely to develop Alzheimer's disease than men. The age refers to the actual age calculated at the time of examination with the actual time of birth as the starting point, and it is known that the likelihood of amyloid-beta deposition increases with age, and especially, Alzheimer's disease mainly occurs in the elderly aged 65 years or older. The education level means the entire level of education including formal education courses, and in the case of dropouts, the level of education was calculated excluding the year in which the student dropped out. Recently, it has been reported that the risk of dementia increases in the case of low education. Therefore, the sex, age, and education level of an individual may be significant variables in predicting the likelihood of brain amyloid-beta deposition.

Step b) is a process of measuring the level of a biomarker present in a biological sample.

The biological sample refers to a sample collected from an individual and usable for predicting the likelihood of brain amyloid-beta deposition, and may be, for example, blood, cerebrospinal fluid, tears, saliva, urine, or the like. In the present invention, it is preferable to use blood, saliva, oral mucosa, or hair, which is relatively painless during the collection process and inexpensive, compared to cerebrospinal fluid.

According to one embodiment of the present invention, the biological sample may be one selected from the group consisting of whole blood, plasma, saliva, oral mucosa, and hair.

The biomarker is a substance that is generally detectable in a biological sample, and examples thereof include all organic biomolecules such as polypeptides, proteins, nucleic acids, genes, lipids, glycolipids, glycoproteins, and sugars, which can indicate biological changes. The present invention is characterized in that the APOE $\varepsilon4$ genotype and the SNP genotype are used as biomarkers.

The APOE gene is classified into three alleles, called $\varepsilon2$ (cys112, cys158), $\varepsilon3$ (cys112, arg158), and $\varepsilon4$ (arg112, arg158), and everyone inherits one APOE allele from each parent. Thus, the genotype of an individual is determined as $\varepsilon2/\varepsilon2$, $\varepsilon2/\varepsilon3$, $\varepsilon2/\varepsilon4$, $\varepsilon3/\varepsilon3$, $\varepsilon3/\varepsilon4$, or $\varepsilon4/\varepsilon4$. Among them, the $\varepsilon4$ allele is found in about 20% of the population and is known to increase the risk of developing Alzheimer's dementia, and thus APOE ε4 is a risk factor highly associated with the development of AD. Therefore, possession of the APOE ε4 genotype can be a significant variable in predicting the likelihood of brain amyloid-beta deposition, and the risk of brain amyloid-beta deposition can increase depending on the number of APOE ε4 alleles.

According to an embodiment of the present invention, when the likelihood of brain amyloid-beta deposition in Koreans is predicted by combining the APOE ε4 genotype with individual characteristics for Koreans, the Area Under Curve (AUC) value can be improved.

Here, AUC means the total area under the ROC curve calculated through ROC analysis, and it is known that the AUC value is 0.5 for a random model and converges to 1 as the predictive performance of the model improves.

The SNP refers to the existence of two or more alleles at one locus, and means that only a single base in the polymorphic site differs. In the present invention, the SNP genotype may be at least one selected from the group consisting of rs73375428, rs6978259, rs2903923, rs3828947, rs6958464 and rs11983537, which were confirmed to be associated with brain amyloid-beta deposition through GWAS analysis performed on the Korean population. Such rs73375428, rs6978259, rs2903923, rs3828947, rs6958464 and rs11983537 are located in the intron of the coiled-coil domain containing 146 (CCDC146) gene in human chromosome 7, show high linkage disequilibrium (LD: $r^2 > 0.75$) with each other, and are located at locus 76907550, locus 76909167, locus 76907750, locus 76908199, locus 76909035, and locus 76908690 of chromosome 7, respectively. These six SNPs have not previously been reported for their association with brain amyloid-beta deposition, and their relationship with brain amyloid-beta deposition in Koreans has also not been reported.

In addition, according to one embodiment of the present invention, AUC can be improved when the likelihood of brain amyloid-beta deposition is predicted by combining individual characteristics and the SNP genotype together with the APOE ε4 genotype.

Step c) is a process of preparing a brain amyloid-beta deposition prediction model by combining individual characteristics, APOE ε4 genotype and SNP genotype.

According to one embodiment of the present invention, step c) may be performed by setting individual characteristics, APOE ε4 genotype, and SNP genotype as independent variables, setting specific coefficients for them as dependent variables, and preparing a brain amyloid-beta deposition prediction model through statistical analysis.

According to one embodiment of the present invention, the statistical analysis may be performed using an analysis method selected from the group consisting of linear or nonlinear regression analysis method, linear or nonlinear classification analysis method, ANOVA, neural network analysis method, deep neural network analysis method, genetic analysis method, support vector machine analysis method, hierarchical analysis or clustering analysis method, hierarchical algorithm or Kernel principal components analysis method using a decision tree, Markov Blanket analysis method, recursive feature elimination or entropy-based recursive feature elimination analysis method, forward floating search or backward floating search analysis method, and combinations thereof.

According to an embodiment of the present invention, a brain amyloid-beta deposition prediction model was prepared by analyzing the relationship between individual characteristics, APOE ε4 genotype and SNP genotype, set as independent variables, and each specific coefficient set as dependent variables, by multivariate logistic regression analysis.

According to one embodiment of the present invention, the brain amyloid-beta deposition prediction model may be represented by Equation 1 below.

[Equation 1]

Aβ deposition = -0.90 + (0.0026*age) + (0.164*sex) + (0.0177*education level) + (1.653*APOE ε4 genotype) + (α*SNP genotype)

(In the Equation 1,
α is -0.612 when SNP is rs6978259, -0.652 when SNP is rs6958464, -0.658 when SNP is rs11983537, -0.571 when SNP is rs3828947, -0.612 when SNP is rs2903923, and -0.633 when SNP is rs73375428.)

According to one embodiment of the present invention, when the likelihood of brain amyloid-beta deposition is predicted by combining individual characteristics, APOE ε4 genotype, and SNP genotype, AUC can be improved. In this case, the AUC in the brain amyloid-beta deposition prediction model is 0.74 or more regardless of the type of SNP (rs6978259, 0.748; rs6958464, 0.749; rs11983537, 0.749; rs3828947, 0.751; rs2903923, 0.748; and rs73375428, 0.749), suggesting that the accuracy is higher than that when only individual characteristics are used alone (0.506) or that when individual characteristics and APOE ε4 genotype are used in combination (0.723).

It can be said that the higher the value derived from the brain amyloid-beta deposition prediction model, the higher the likelihood of brain amyloid-beta deposition in the subject to be tested.

In addition, another aspect of the present invention provides a composition for predicting the likelihood of brain amyloid-beta deposition, the composition containing an agent capable of detecting APOE ε4 genotype and at least one SNP genotype selected from the group consisting of rs73375428, rs6978259, rs2903923, rs3828947, rs6958464, and rs11983537.

In the present invention, the expression "composition for predicting the likelihood of brain amyloid-beta deposition" refers to a composition for a series of actions of predicting brain amyloid-beta deposition by detecting nucleotide sequences including APOE ε4 genotype and SNP genotype. Using this composition, the risk of developing a disease by brain amyloid-beta deposition or whether a disease has been developed by brain amyloid-beta deposition may be diagnosed at an early stage.

Here, the APOE ε4 genotype and the six SNP genotypes are the same as described above.

The agent is capable of detecting biomarkers, that is, the APOE ε4 genotype and the SNP genotype, for predicting the likelihood of brain amyloid-beta deposition.

According to one embodiment of the present invention, the agent may be at least one selected from the group consisting of a primer set, a probe, and an antisense oligonucleotide, which specifically bind to a nucleotide sequence including APOE ε4 genotype or SNP genotype. In the present invention, the agent is preferably a primer set designed to specifically amplify a specific region of a gene based on nucleic acid sequence information of genes, or a probe.

The term "specifically bind" means that the binding affinity to a target substance is superior to the binding affinity to other substances to the extent that the presence or absence of the target substance can be detected by binding.

The primer set refers to a combination of primers that are short-stranded RNA or DNA sequences that recognize all or part of a nucleotide sequence including the APOE ε4 genotype or SNP genotype. For example, the primer set may be a primer set of any combination of forward and reverse primers capable of binding to a nucleotide sequence including the APOE ε4 genotype or SNP genotype. Since the nucleic acid sequence of the primer is a sequence that does not match the non-target sequence present in the sample, it may amplify only the target gene sequence containing the complementary primer binding site, and may give high specificity when the primer does not cause non-specific amplification.

The probe refers to a substance capable of specifically binding to a target substance to be detected in a sample, and capable of specifically detecting the presence of the target substance in the sample through the binding. The constituent material of the probe is a material capable of specifically binding to the target substance, and examples thereof include, but are not limited to, peptide nucleic acid (PNA), locked nucleic acid (LNA), peptides, polypeptides, proteins, RNA, DNA, and the like.

Still another aspect of the present invention provides a kit for predicting the likelihood of brain amyloid-beta deposition, the kit comprising the composition.

In the present invention, the expression "kit for predicting the likelihood of brain amyloid-beta deposition" refers to a substance capable of predicting the likelihood of brain amyloid-beta deposition through a biological sample collected from a test subject. Using the kit, the risk of developing a disease by brain amyloid-beta deposition or whether a disease has been developed by brain amyloid-beta deposition may be diagnosed at an early stage in a rapid and convenient manner. Examples of the kit include, but are not limited to, a polymerase chain reaction (PCR) kit, a DNA chip kit, etc. In addition, the kit may further comprise one or more other compositions, solutions or devices suitable for assay.

According to one embodiment of the present invention, the kit may comprise essential elements necessary for performing polymerase chain reaction (PCR). In this case, the kit may comprise, in addition to a primer set that specifically binds to a nucleotide sequence including the APOE ε4 genotype or at least one SNP genotypes selected from the group consisting of rs73375428, rs6978259, rs2903923, rs3828947, rs6958464 and rs11983537, a test tube or other appropriate container, a reaction buffer (which may have various pHs and magnesium concentrations depending on conditions), deoxynucleotides (dNTPs), enzymes such as Taq polymerase and reverse transcriptase, DNase and RNAse inhibitors, DEPC-treated water, sterilized water, etc.

According to one embodiment of the present invention, the kit may comprise essential elements necessary for performing DNA chip assay. In this case, the kit may comprise a substrate to which a cDNA or oligonucleotide corresponding to a nucleotide sequence including the APOE ε4 genotype or at least one SNP genotype selected from the group consisting of rs73375428, rs6978259, rs2903923, rs3828947, rs6958464 and rs11983537 or a fragment of the nucleotide sequence is attached, and a reagent, an agent, an enzyme, and the like for producing a fluorescently labeled probe. In addition, the substrate may comprise a cDNA or oligonucleotide corresponding to a control gene or a fragment thereof.

**Advantageous Effects**

The method of providing information for predicting the likelihood of brain amyloid-beta deposition according to the present invention may effectively screen individuals with a high likelihood of brain amyloid-beta deposition at an early stage, thereby providing an appropriate method of inhibiting the development of a disease caused by brain amyloid-beta deposition or treating an individual having the disease.

**Brief Description of Drawings**

FIG. 1 is a quantile-quantile plot of P values according to an Example of the present invention.

FIG. 2 is a graph showing SNPs located on chromosomes 19 and 7 in the whole genome and significantly associated with amyloid-beta deposition, according to an Example of the present invention.

FIG. 3 is a graph showing the chromosomal location of rs6978259 according to an Example of the present invention.

FIG. 4 shows amyloid positron emission tomography (PET) images of subjects for voxel-based morphometry analysis according to an Example of the present invention. Specifically, (A) depicts images showing that Aβ deposition decreases in a subject with the rs6978259 allele, and (B) depicts images showing that Aβ deposition increases in a subject with the APOE ε4 allele.

FIG. 5 shows AUC values of datasets for development and validation of models for rs6978259 according to an Example of the present invention.

FIG. 6 shows AUC values of datasets for development and validation of models for rs6958464 according to an Example of the present invention.

FIG. 7 shows AUC values of datasets for development and validation of models for rs11983537 according to an Example of the present invention.

FIG. 8 shows AUC values of datasets for development and validation of models for rs3828947 according to an Example of the present invention.

FIG. 9 shows AUC values of datasets for development and validation of models for rs2903923 according to an Example of the present invention.

FIG. 10 shows AUC values of datasets for development and validation of models for rs73375428 according to an Example of the present invention.

**Mode for Invention**

Hereinafter, the present invention will be described in more detail. However, these descriptions are provided for illustrative purposes only to help understand the present invention, and the scope of the present invention is not limited by these exemplary descriptions.

**Example 1. Development of model for predicting likelihood of brain amyloid-beta deposition**

**1-1. Recruitment of participants**

For a dataset development of a model, a total of 1,214 Koreans were recruited from 14 domestic hospitals from January 2013 to July 2019. Among them, 923 participants were recruited from the Samsung Medical Center, 201 participants were recruited from a multicenter study of the Korean Brain Aging Study for the Early Diagnosis and Prediction of AD (KBASE-V), and 90 participants were recruited from a multicenter study of Clinical Research Platform based on Dementia Cohort.

For a dataset for validation, data from 306 Korean participants were collected from the Biobank of the Chronic Cerebrovascular Disease Consortium, recruited from 2016 to 2018. This was part of the ongoing Biobank Innovation for chronic Cerebrovascular disease With ALZheimer's disease Study (BICWALZS) and the Center for Convergence Research of Neurological Disorders.

Among the recruited test subjects, the present inventors included participants (i) who were diagnosed with amnestic mild cognitive impairment (aMCI), AD dementia (ADD), or were cognitively unimpaired (CU) based on detailed neuropsychological tests, and (ii) who underwent amyloid PET imaging, and excluded participants who had a causative genetic mutation for AD, such as PSEN1, PSEN2, and APP. Quality control (QC) was performed as described later, and for data for model development, a total of 1,190 participants (383 with CU, 330 with aMCI, and 477 with ADD) were used, and for data for validation, a total of 284 participants (46 with CU, 167 with aMCI, and 71 with ADD) were used. All participants provided written informed consent, and the study was approved by the Institutional Review Board of each center.

As a result, the baseline demographic characteristics of the two datasets (data for model development and data for verification) are shown in Table 1 below. Participants for model development were younger and had a higher proportion of females than data for validation. As expected, in both datasets, amyloid-beta (Aβ) positive participants confirmed by PET had higher rates of aMCI and AD than Aβ negative participants. In addition, in the data for validation, Aβ-positive participants were older than Aβ-negative participants.

[Table 1]

| | Data for model development | | | |
|---|---|---|---|---|
| Demographic characteristics | Total (n=1,190) | Aβ negative (n=561) | Aβ positive (n=629) | P value* |
| Age, year (SD) | 70.07 (8.75) | 70.06 (8.16) | 70.07 (9.25) | 0.990 |
| Female, n (%) | 680 (57.1) | 310 (55.3) | 370 (58.8) | 0.215 |
| Education, year (SD) | 11.02 (4.86) | 10.89 (5.04) | 11.13 (4.70) | 0.390 |
| Diagnosis, n (%)<br>CU<br>aMCI<br>ADD | <br>383 (32.2)<br>330 (27.7)<br>477 (40.1) | <br>326 (58.1)<br>172 (30.7)<br>63 (11.2) | <br>57 (9.1)<br>158 (25.1)<br>414 (65.8) | <0.001 |
| | Data for validation | | | |
| Demographic characteristics | Total (n=284) | Aβ negative (n=180) | Aβ positive (n=104) | P value* |
| Age, year (SD) | 72.67 (7.32) | 71.76 (7.31) | 74.25 (7.10) | 0.006 |
| Female, n (%) | 184 (64.8) | 122 (67.8) | 62 (59.6) | 0.165 |
| Education, year (SD) | 8.34 (5.21) | 7.77 (5.19) | 9.11 (5.15) | 0.050 |
| Diagnosis, n (%) | | | | <0.001 |
| CU<br>aMCI<br>ADD | 46 (16.2)<br>167 (58.8)<br>71 (25.0) | 43 (23.9)<br>125 (69.4)<br>12 (6.7) | 3 (2.9)<br>42 (40.4)<br>59 (56.7) | |
| *: P value was calculated by comparing Aβ negative and Aβ positive participants.<br>P values calculated by comparing the data for model development and the data for validation were <0.001, 0.019, <0.001, and <0.001 for age, sex, education, and diagnosis, respectively.<br>Student's t test and chi-squared test were used for continuous and categorical variables, respectively. | | | | |

**1-2. Genotyping and imputation**

Participants were genotyped using the Illumina Asian Screening Array BeadChip (Illumina, USA) for data for model development and Affymetrix customized Korean chips (Affymetrix, CA, USA) for data for model validation, and SNP markers were analyzed. The present inventors conducted QC using PLINK software (version 1.9). Participants were excluded based on the following criteria: (i) call rate < 95%, (ii) mismatch between reported and genetically inferred sex, (iii) deviation from each population parameter, (iv) excess heterozygosity rate (5 standard deviation from the mean), and (v) in cases of related pairs (identified with identity by descent (IBD) $\geq$ 0.125) within and between the datasets for model development and for model validation.

SNPs were excluded based on the following criteria: (i) call rate < 98%, (ii) minor allele frequency (MAF) < 1%, and (iii) a p value < $1.0 \times 10^{-6}$ for the Hardy-Weinberg equilibrium test.

After QC, genome-wide imputation was performed using the Minimac4 software with all available reference haplotypes from HRC-r1.1 on the University of Michigan Imputation Server. For post-imputation QC, the present inventors excluded SNPs based on the following criteria: (i) poor imputation quality ($R^2 \leq 0.8$), and (ii) MAF $\leq$ 1%. Finally, 4,906,407 SNPs were analyzed.

**1-3. Aβ PET acquisition and voxel-based morphometry analysis**

Amyloid-beta (Aβ) PET images were obtained using a Discovery STE PET/CT scanner (GE Medical Systems, USA). PET images were acquired for 20 min, starting at 90 min after intravenous injection of either $^{18}$F-florbetaben or $^{18}$F-flutemetamol. Aβ positivity or negativity was determined using visual assessments for FBB and FMM PET. A subset of participants in the cohort for model development (n = 824) had Aβ PET data available for voxel-based morphometry (VBM) analysis.

For VBM analysis, the present inventors performed the following preprocessing using Statistical Parametric Mapping software version 12 (SPM, http://www.fil.ion/uc.ac.uk/spm) running on MATLAB (MathWorks 2014b): (1) co-registration of PET to T1-weighted structural MRI, (2) structural MRI segmentation and calculation of transformation matrix, (3) normalization of PET to a Montreal Neurological Institute (MNI) space, and (4) spatial smoothing with a Gaussian kernel of 8-mm full width at half maximum.

To calculate the standardized uptake value ratio (SUVR) for each PET image, the present inventors used two reference regions (the cerebellar cortex for FBB and pons for FMM). The masks of reference regions were obtained from the GAAIN website (http://www.GAAIN.org).

**1-4. Statistical analysis**

**① GWAS analysis**

Logistic regression analysis was performed to determine the association between SNPs and Aβ positivity in a state in which age, gender, and population subgroup effects were adjusted, and the additive model, coded as 0, 1, and 2 according to the number of minor alleles, was used. In order to adjust for population subgroup effects, principal component analysis was performed on the genetic data, and the first, second, and third principal components were used as correction variables. Reported p values were two-tailed, and the present inventors defined a p value less than $5.0 \times 10^{-8}$ as being statistically significant and a p value less than $1.0 \times 10^{-5}$ as being statistically suggestive based on previous studies. Genomic inflation was evaluated by dividing the median of chi-squared statistics derived from GWAS analysis by the median (0.456) of a chi-square distribution with a degree of freedom of 1. For validation analysis, considering that the effect is expected to occur in the same direction as the result of the dataset for model development, one-tailed P values were reported. To check if SNPs were associated with Aβ positivity independent of APOE genotype, the present inventors performed conditional analysis by further adjusting for APOE genotype.

Furthermore, the present inventors performed P-value based meta-analysis and calculated the summary effect size by applying weights reflecting the standard errors from the study specific effect sizes.

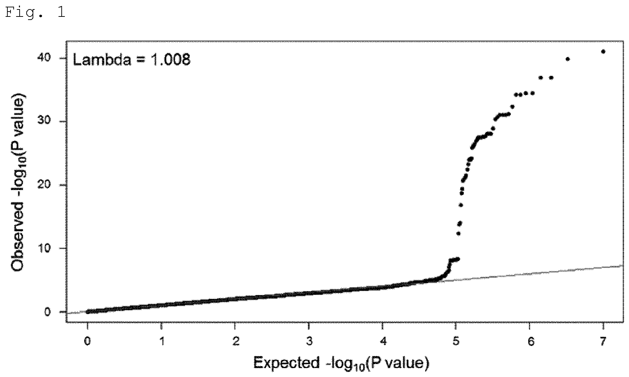

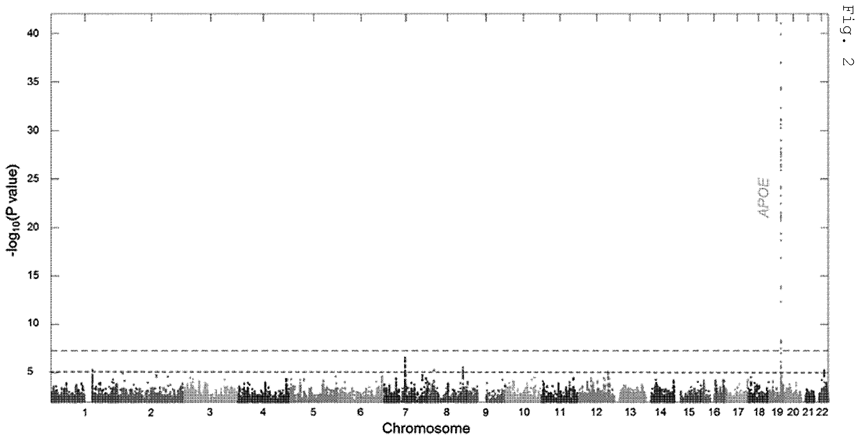

As a result, referring to FIG. 1, a quantile-quantile (Q-Q) plot of p values revealed no genomic inflation (λ = 1.008). Referring to FIG. 2, in the data for model development, 61 genome-wide significant SNPs on chromosome 19 (P value $< 5 \times 10^{-8}$) were identified (see Table 2 below) . However, all significant SNPs fell within the 500-kb region surrounding APOE gene and lost genome-wide significance when the APOE ε4 allele was adjusted. Outside of the APOE region, 38 SNPs on chromosomes 1, 7, 8, 12, and 22 showed genome-wide suggestive significance (P value < $1.0 \times 10^{-5}$) (see Table 3 below). In validation analysis, among 38 suggestive SNPs, 6 SNPs (rs73375428, rs6978259, rs2903923, rs3828947, rs6958464 and rs11983537) on chromosome 7 showed a significant association in the same direction as the results obtained from the data for model development. Two SNPs (rs6958464 and rs11983537) among the six SNPs were directly genotyped and the remaining SNPs (rs73375428, rs6978259, rs2903923 and rs3828947) were analyzed by imputation. In particular, two of the six SNPs (rs73375428 and rs2903923) showed genome-wide significant associations in the meta-analysis of the datasets for model development and validation (see Tables 4 and 5 below). When the effect of the APOE ε4 allele was adjusted, all of the six SNPs showed P values at genome-wide suggestive levels, and the associations were verified in the data for validation. As shown in Table 6 below, the identified six SNPs showed high linkage disequilibrium (LD: $r^2 > 0.75$) with each other, and thus rs6978259 out of the six SNPs was used as a representative for subsequent analysis.

[Table 2]

| SNP | Chromos ome | Base pairs (bp) | Effective allele | OR | P value | P value* |
|---|---|---|---|---|---|---|
| rs8112196 | 19 | 45325309 | A | 0.632 | $9.12 \times 10^{-8}$ | 0.086 |
| rs6509172 | 19 | 45325391 | T | 0.616 | $7.06 \times 10^{-9}$ | 0.044 |
| rs 1871045 | 19 | 45326768 | T | 0.613 | $6.32 \times 10^{-9}$ | 0.056 |
| rs73050216 | 19 | 45367502 | C | 0.527 | $1.93 \times 10^{-14}$ | 0.049 |

(continued)

| SNP | Chromos ome | Base pairs (bp) | Effective allele | OR | P value | P value* |
|---|---|---|---|---|---|---|
| rs41290098 | 19 | 45370278 | T | 0.547 | $4.59 \times 10^{-13}$ | 0.165 |
| rs 12610605 | 19 | 45370838 | G | 1.917 | $1.21 \times 10^{-14}$ | 0.354 |
| rs404935 | 19 | 45372794 | A | 3.472 | $1.07 \times 10^{-29}$ | 0.193 |
| rs395908 | 19 | 45373565 | A | 3.332 | $2.85 \times 10^{-28}$ | 0.451 |
| rs34278513 | 19 | 45378144 | T | 2.868 | $1.12 \times 10^{-21}$ | 0.515 |
| rs412776 | 19 | 45379516 | A | 3.601 | $5.02 \times 10^{-31}$ | 0.149 |
| rs3865427 | 19 | 45380961 | A | 3.122 | $5.34 \times 10^{-24}$ | 0.314 |
| rs6859 | 19 | 45382034 | A | 2.110 | $1.39 \times 10^{-17}$ | 0.654 |
| rs3852860 | 19 | 45382966 | C | 2.875 | $5.43 \times 10^{-27}$ | 0.113 |
| rs3852861 | 19 | 45383061 | G | 2.850 | $1.19 \times 10^{-26}$ | 0.135 |
| rs71352237 | 19 | 45383079 | C | 3.288 | $9.49 \times 10^{-25}$ | 0.493 |
| rs34224078 | 19 | 45383115 | G | 3.288 | $9.49 \times 10^{-25}$ | 0.493 |
| rs35879138 | 19 | 45383139 | A | 3.307 | $6.24 \times 10^{-25}$ | 0.430 |
| rs 166907 | 19 | 45386855 | G | 6.140 | $3.09 \times 10^{-8}$ | 0.231 |
| rs12972156 | 19 | 45387459 | G | 3.830 | $7.66 \times 10^{-29}$ | 0.427 |
| rs12972970 | 19 | 45387596 | A | 3.830 | $7.66 \times 10^{-29}$ | 0.427 |
| rs34342646 | 19 | 45388130 | A | 3.830 | $7.66 \times 10^{-29}$ | 0.427 |
| rs283815 | 19 | 45390333 | G | 3.338 | $6.85 \times 10^{-32}$ | 0.298 |
| rs6857 | 19 | 45392254 | T | 3.681 | $5.38 \times 10^{-28}$ | 0.261 |
| rs71352238 | 19 | 45394336 | C | 3.748 | $2.03 \times 10^{-28}$ | 0.368 |
| rs184017 | 19 | 45394969 | G | 3.289 | $2.24 \times 10^{-31}$ | 0.396 |
| rs157580 | 19 | 45395266 | G | 0.462 | $2.12 \times 10^{-19}$ | 0.154 |
| rs2075650 | 19 | 45395619 | G | 3.748 | $2.03 \times 10^{-28}$ | 0.368 |
| rs157581 | 19 | 45395714 | C | 2.820 | $3.31 \times 10^{-27}$ | 0.966 |
| rs34404554 | 19 | 45395909 | G | 3.716 | $3.22 \times 10^{-28}$ | 0.293 |
| rs11556505 | 19 | 45396144 | T | 3.716 | $3.22 \times 10^{-28}$ | 0.293 |
| rs157582 | 19 | 45396219 | T | 3.326 | $8.93 \times 10^{-32}$ | 0.373 |
| rs59007384 | 19 | 45396665 | T | 2.802 | $3.38 \times 10^{-23}$ | 0.965 |
| rs157583 | 19 | 45396673 | T | 6.677 | $5.65 \times 10^{-9}$ | 0.117 |
| rs157587 | 19 | 45398206 | G | 6.571 | $7.71 \times 10^{-9}$ | 0.122 |
| rs205909 | 19 | 45400775 | G | 6.677 | $5.65 \times 10^{-9}$ | 0.117 |
| rs491153 | 19 | 45402368 | T | 6.677 | $5.65 \times 10^{-9}$ | 0.117 |
| rs490243 | 19 | 45402470 | T | 6.677 | $5.65 \times 10^{-9}$ | 0.117 |
| rs417357 | 19 | 45403119 | T | 6.775 | $4.24 \times 10^{-9}$ | 0.114 |
| rs394819 | 19 | 45404579 | T | 6.775 | $4.24 \times 10^{-9}$ | 0.114 |
| rs405697 | 19 | 45404691 | G | 2.299 | $1.86 \times 10^{-21}$ | 0.170 |
| rs10119 | 19 | 45406673 | A | 4.948 | $1.21 \times 10^{-40}$ | 0.967 |
| rs435380 | 19 | 45407118 | A | 6.576 | $7.63 \times 10^{-9}$ | 0.133 |

(continued)

| SNP | Chromos ome | Base pairs (bp) | Effective allele | OR | P value | P value* |
|---|---|---|---|---|---|---|
| rs446037 | 19 | 45407437 | T | 6.577 | $7.59 \times 10^{-9}$ | 0.147 |
| rs434132 | 19 | 45407720 | G | 6.577 | $7.59 \times 10^{-9}$ | 0.147 |
| rs439382 | 19 | 45408475 | G | 6.573 | $7.68 \times 10^{-0}$ | 0.107 |
| rs440446 | 19 | 45409167 | G | 2.318 | $3.06 \times 10^{-22}$ | 0.318 |
| rs769449 | 19 | 45410002 | A | 4.601 | $4.83 \times 10^{-33}$ | 0.223 |
| rs429358 | 19 | 45411941 | C | 5.275 | $9.03 \times 10^{-42}$ | 0.601 |
| rs75627662 | 19 | 45413576 | T | 3.392 | $1.55 \times 10^{-28}$ | 0.790 |
| rs439401 | 19 | 45414451 | C | 2.295 | $5.58 \times 10^{-22}$ | 0.279 |
| rs10414043 | 19 | 45415713 | A | 4.285 | $8.20 \times 10^{-32}$ | 0.216 |
| rs7256200 | 19 | 45415935 | T | 4.285 | $8.20 \times 10^{-32}$ | 0.216 |
| rs483082 | 19 | 45416178 | T | 4.024 | $1.01 \times 10^{-37}$ | 0.213 |
| rs584007 | 19 | 45416478 | G | 2.284 | $8.19 \times 10^{-22}$ | 0.310 |
| rs438811 | 19 | 45416741 | T | 4.024 | $1.01 \times 10^{-37}$ | 0.213 |
| rs5117 | 19 | 45418790 | C | 3.316 | $1.17 \times 10^{-27}$ | 0.921 |
| rs12721046 | 19 | 45421254 | A | 4.497 | $6.23 \times 10^{-35}$ | 0.209 |
| rs12721051 | 19 | 45422160 | G | 4.493 | $5.81 \times 10^{-35}$ | 0.214 |
| rs56131196 | 19 | 45422846 | A | 4.517 | $3.73 \times 10^{-35}$ | 0.291 |
| rs4420638 | 19 | 45422946 | G | 4.517 | $3.73 \times 10^{-35}$ | 0.291 |
| rs157595 | 19 | 45425460 | G | 2.197 | $4.31 \times 10^{-20}$ | 0.520 |
| *: P values are statistical values of logistic regression analysis adjusted for the effect of the APOE $\varepsilon$4 allele | | | | | | |

[Table 3]

| SNP | Chromo some | Base pairs (bp) | Effective allele | Data for model development (n=1190) | | Data for validation (n=284) | |
|---|---|---|---|---|---|---|---|
| | | | | OR | P value | OR | P value* |
| rs73375428 | 7 | 76907550 | G | 0.519 | $2.71 \times 10^{-7}$ | 0.549 | 0.020 |
| rs6978259 | 7 | 76909167 | C | 0.521 | $4.62 \times 10^{-7}$ | 0.566 | 0.030 |
| rs2903923 | 7 | 76907750 | G | 0.528 | $5.15 \times 10^{-7}$ | 0.539 | 0.016 |
| rs3828947 | 7 | 76908199 | C | 0.528 | $5.15 \times 10^{-7}$ | 0.546 | 0.018 |
| rs6958464 | 7 | 76909035 | T | 0.525 | $6.28 X 10^{-'}$ | 0.555 | 0.029 |
| rs11983537 | 7 | 76908690 | T | 0.557 | $7.58 \times 10^{-7}$ | 0.539 | 0.013 |
| rs112599253 | 7 | 76931677 | T | 0.561 | $1.56 \times 10^{-7}$ | 0.723 | 0.107 |
| rs79761449 | 7 | 76925493 | T | 0.564 | $2.50 \times 10^{-7}$ | 0.723 | 0.107 |
| rs6971106 | 7 | 76929191 | T | 0.564 | $2.50 \times 10^{-7}$ | 0.723 | 0.107 |
| rs113931965 | 7 | 76930080 | T | 0.564 | $2.50 \times 10^{-6}$ | 0.723 | 0.107 |
| rs113014884 | 7 | 76930314 | C | 0.564 | $2.50 \times 10^{-6}$ | 0.723 | 0.107 |
| rs58731747 | 7 | 76935492 | A | 0.568 | $2.75 \times 10^{-6}$ | 0.706 | 0.091 |

(continued)

| SNP | Chromosome | Base pairs (bp) | Effective allele | Data for model development (n=1190) | | Data for validation (n=284) | |
|---|---|---|---|---|---|---|---|
| | | | | OR | P value | OR | P value* |
| rs1077236 | 8 | 130640501 | T | 1.612 | $2.85 \times 10^{-6}$ | 0.993 | 0.488 |
| rs10092679 | 8 | 130682158 | T | 1.580 | $5.33 \times 10^{-6}$ | 0.941 | 0.390 |
| rs1196470 | 1 | 151706235 | A | 0.669 | $5.43 \times 10^{-6}$ | 0.992 | 0.484 |
| rs4872051 | 8 | 22945345 | G | 0.687 | $5.66 \times 10^{-6}$ | 0.827 | 0.135 |
| rs140005 | 22 | 36943544 | A | 1.488 | $5.76 \times 10^{-6}$ | 1.035 | 0.424 |
| rs140008 | 22 | 36944165 | T | 1.488 | $5.76 \times 10^{-6}$ | 1.035 | 0.424 |
| rs140010 | 22 | 36944356 | C | 1.488 | $5.76 \times 10^{-6}$ | 1.035 | 0.424 |
| rs68055908 | 8 | 130667115 | T | 1.579 | $5.85 \times 10^{-6}$ | 0.976 | 0.455 |
| rs59674745 | 7 | 76913192 | C | 0.511 | $5.86 \times 10^{-6}$ | 0.831 | 0.308 |
| rs6470745 | 8 | 130641921 | G | 1.576 | $6.36 \times 10^{-6}$ | 1.002 | 0.497 |
| rs6985032 | 8 | 130671748 | T | 1.561 | $7.46 \times 10^{-6}$ | 0.958 | 0.418 |
| rs4295627 | 8 | 130685457 | G | 1.569 | $7.61 \times 10^{-6}$ | 0.910 | 0.333 |
| rs28572791 | 8 | 130641782 | C | 1.569 | $7.92 \times 10^{-6}$ | 1.033 | 0.436 |
| rs2868782 | 7 | 76907862 | A | 0.517 | $8.06 \times 10^{-6}$ | 0.781 | 0.259 |
| rs7306151 | 12 | 110092612 | C | 0.619 | $8.24 \times 10^{-6}$ | 0.903 | 0.316 |
| rs6970348 | 7 | 76929400 | A | 0.610 | $8.27 \times 10^{-6}$ | 0.780 | 0.153 |
| rs140018 | 22 | 36946330 | C | 1.479 | $8.97 \times 10^{-6}$ | 0.985 | 0.467 |
| rs140019 | 22 | 36946491 | G | 1.479 | $8.97 \times 10^{-6}$ | 0.985 | 0.467 |
| rs140011 | 22 | 36945022 | G | 1.479 | $9.02 \times 10^{-6}$ | 0.990 | 0.479 |
| rs140012 | 22 | 36945289 | C | 1.479 | $9.02 \times 10^{-6}$ | 0.990 | 0.479 |
| rs140014 | 22 | 36945641 | C | 1.479 | $9.02 \times 10^{-6}$ | 0.990 | 0.479 |
| rs140016 | 22 | 36945847 | C | 1.479 | $9.02 \times 10^{-6}$ | 0.990 | 0.479 |
| rs1400 17 | 22 | 36945947 | A | 1.479 | $9.02 \times 10^{-6}$ | 0.990 | 0.479 |
| rs144786745 | 8 | 130658990 | T | 1.569 | $9.23 \times 10^{-6}$ | 1.039 | 0.427 |
| rs4766712 | 12 | 114491139 | T | 1.511 | $9.40 \times 10^{-6}$ | 0.870 | 0.251 |
| rs5022680 | 8 | 130685516 | T | 1.562 | $9.42 \times 10^{-6}$ | 0.923 | 0.356 |

*: P value is statistical values of the logistic regression analysis performed on date for validation.

[Table 4]

| SNP | Chromosome: base pairs (bp) | Effective allele |
|---|---|---|
| rs73375428 | 7:76907550 | G |
| rs6978259 | 7:76909167 | C |
| rs2903923 | 7:76907750 | G |
| rs3828947 | 7:76908199 | C |
| rs6958464 | 7:76909035 | T |

(continued)

| SNP | Chromosome: base pairs (bp) | Effective allele |
|---|---|---|
| rs11983537 | 7:76908690 | T |

[Table 5]

| SNP | Analysis 1 | | | | | |
|---|---|---|---|---|---|---|
| | Data for model development | | Data for validation | | Meta-analysis | |
| | OR | P value | OR | P value | OR | P value |
| rs73375428 | 0.519 | $2.71 \times 10^{-7}$ | 0.550 | 0.020 | 0.526 | $3.35 \times 10^{-8}$ |
| rs6978259 | 0.522 | $4.62 \times 10^{-'}$ | 0.566 | 0.030 | 0.521 | $8.54 \times 10^{-8}$ |
| rs2903923 | 0.529 | $5.15 \times 10^{-7}$ | 0.539 | 0.016 | 0.536 | $4.97 \times 10^{-8}$ |
| rs3828947 | 0.529 | $5.15 \times 10^{-7}$ | 0.547 | 0.018 | 0.536 | $5.59 \times 10^{-8}$ |
| rs6958464 | 0.526 | $6.28 \times 10^{-7}$ | 0.555 | 0.028 | 0.525 | $4.06 \times 10^{-7}$ |
| rs11983537 | 0.558 | $7.58 \times 10^{-7}$ | 0.539 | 0.013 | 0.563 | $5.92 \times 10^{-8}$ |
| SNP | Analysis 2 | | | | | |

| | Data for model development | | Data for validation | | Meta-analysis | |
|---|---|---|---|---|---|---|
| | OR | P value | OR | P value | OR | P value |
| rs73375428 | 0.535 | $1.23 \times 10^{-5}$ | 0.481 | 0.011 | 0.516 | $8.00 \times 10^{-7}$ |
| rs6978259 | 0.521 | $7.90 \times 10^{-6}$ | 0.515 | 0.020 | 0.541 | $9.08 \times 10^{-7}$ |
| rs2903923 | 0.546 | $2.19 \times 10^{-5}$ | 0.478 | 0.010 | 0.510 | $1.32 \times 10^{-6}$ |
| rs3828947 | 0.546 | $2.19 \times 10^{-5}$ | 0.480 | 0.010 | 0.515 | $1.39 \times 10^{-6}$ |
| rs6958464 | 0.524 | $9.63 \times 10^{-6}$ | 0.484 | 0.014 | 0.521 | $8.18 \times 10^{-7}$ |
| rs11983537 | 0.570 | $1.99 \times 10^{-5}$ | 0.492 | 0.010 | 0.517 | $1.22 \times 10^{-6}$ |
| Analysis 1: Values of logistic regression analysis not adjusted for the effect of APOE $\varepsilon 4$ allele. Analysis 2: Values of logistic regression analysis adjusted for the effect of APOE $\varepsilon 4$ allele. | | | | | | |

[Table 6]

| | rs73375428 | rs2903923 | rs3828947 | rs11983537 | rs6958464 | rs6978259 |
|---|---|---|---|---|---|---|
| rs73375428 | 1 | 1.00 | 1.00 | 0.92 | 0.87 | 0.87 |
| rs2903923 | 1.00 | 1 | 1.00 | 0.92 | 0.87 | 0.87 |
| rs3828947 | 1.00 | 1.00 | 1 | 0.92 | 0.87 | 0.87 |
| rs11983537 | 0.92 | 0.92 | 0.92 | 1 | 0.88 | 0.88 |
| rs6958464 | 0.87 | 0.87 | 0.87 | 0.88 | 1 | 1.00 |
| rs6978259 | 0.87 | 0.87 | 0.87 | 0.88 | 1.00 | 1 |

## ② Effects of newly identified SNPs

After identifying associated SNPs, the present inventors calculated the risk of the six SNPs on Aβ deposition in all participants and at each cognitive level (CU, aMCI, and ADD).

**Next,** the present inventors performed voxel-wise PET image analysis to determine which regional Aβ deposition is associated with SNPs after adjusting for the effects of age, sex, genetic PCs, APOE genotype, and Aβ PET tracer type.

To test the clinical utility of the newly identified SNPs, the present inventors developed multivariable logistic models to predict Aβ positivity in each individual. To evaluate the performance of the logistic model, the present inventors measured the area under curve (AUC) from the receiver operating characteristic (ROC) curve analysis.

For internal validation, the present inventors conducted a 10-fold cross-validation with 100 repeats using the data for model development. The present inventors reported the mean AUC with 95% confidence interval (CI) of the model. As an external validation, parameters estimated from the data for model development were used to calculate the Aβ prediction performance in the data for validation. The present inventors used R software (http://www.rproject.org) and MATLAB for the statistical analyses and results visualization.

As a result, the characteristics of the participants for the number of minor alleles (C) of rs6978259 are shown in Table 7 below.

[Table 7]

|  | C=0 (n=894) | C=1 (n=278) | C=2 (n=18) | P 값* |
|---|---|---|---|---|
| Age, year (SD) | 70.06 (8.74) | 70.01 (8.86) | 71.22 (7.62) | 0.850 |
| Education, year (SD) | 11.21 (4.80) | 10.43 (5.01) | 10.38 (5.00) | 0.054 |
| Diagnosis, n (%) |  |  |  | 0.014 |
| CU | 268 (30.0) | 107 (38.5) | 8 (44.4) |  |
| aMCI | 247 (27.6) | 76 (27.3) | 7 (39.8) |  |
| ADD | 379 (42.4) | 95 (34.2) | 3 (16.7) |  |
| Female, n (%) | 495 (55.4) | 173 (62.2) | 12 (66.7) | 0.093 |
| APOE ε4, n (%) |  |  |  | 0.012 |
| Noncarrier | 511 (57.2) | 187 (67.3) | 13 (72.2) |  |
| Heterozygote | 306 (34.2) | 65 (23.4) | 4 (22.2) |  |
| Homozygote | 77 (8.6) | 26 (9.4) | 1 (5.6) |  |
| Aβ positive, n (%) | 507 (56.7) | 120 (43.2) | 2 (11.1) | <0.001 |
| *: P values were calculated using one-way ANOVA or Chi-square test, as appropriate. | | | | |

As shown in Table 8 below, in the logistic model, the APOE ε4 allele was associated with a 5-fold higher risk of Aβ positivity (odds ratio [OR] = 5.330; 95% CI = 4.188-6.788), and rs6978259 was associated with a 2-fold lower risk of Aβ positivity (OR = 0.521. 95% CI = 0.407-0.670). In the subgroup analysis, the association of rs6978259 with Aβ positivity was significant in the CU and aMCI groups but not in the ADD group, while the association of APOE ε4 was significant across all cognitive states.

[Table 8]

|  | rs6978259 | | APOE ε4 | |
|---|---|---|---|---|
|  | OR (95% CI) | P value | OR (95% CI) | P value |
| Total (n=1190) | 0.521 (0.407-0.670) | <0.001 | 5.330 (4.188-6.788) | <0.001 |
| CU (n=383) | 0.503 (0.256-0.988) | 0.046 | 3.885 (2.307-6.54) | <0.001 |
| aMCI (n=330) | 0.455 (0.281-0.735) | 0.001 | 6.655 (4.101-10.8) | <0.001 |
| ADD (n=477) | 0.667 (0.365-1.219) | 0.188 | 4.272 (2.428-7.516) | <0.001 |

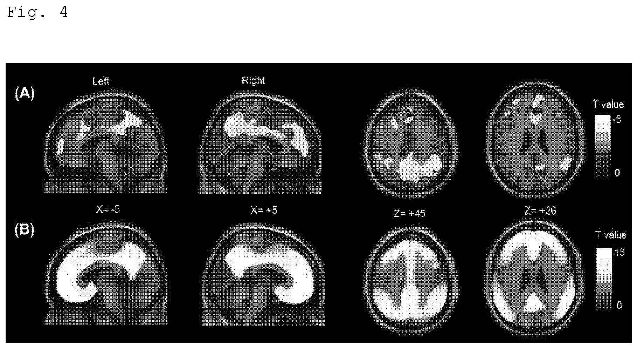

Referring to FIG. 4, in the VBM analysis, APOE ε4 was associated with increased Aβ deposition on the wide cortical areas of the frontal, parietal, and temporal lobes. It was confirmed that rs6978259 was associated with decreased Aβ deposition in the precuneus, lateral parietal, and medial frontal areas, independent of age, sex, education level, APOE ε4, and Aβ PET tracer type.

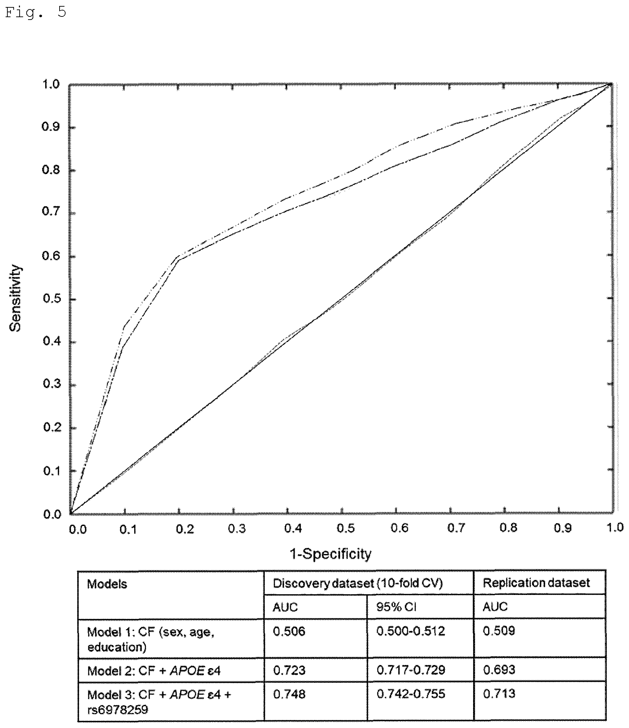

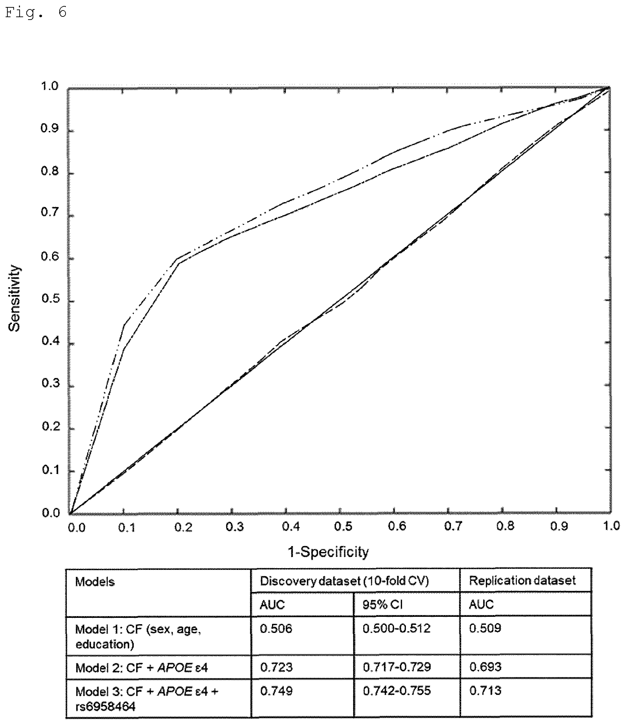

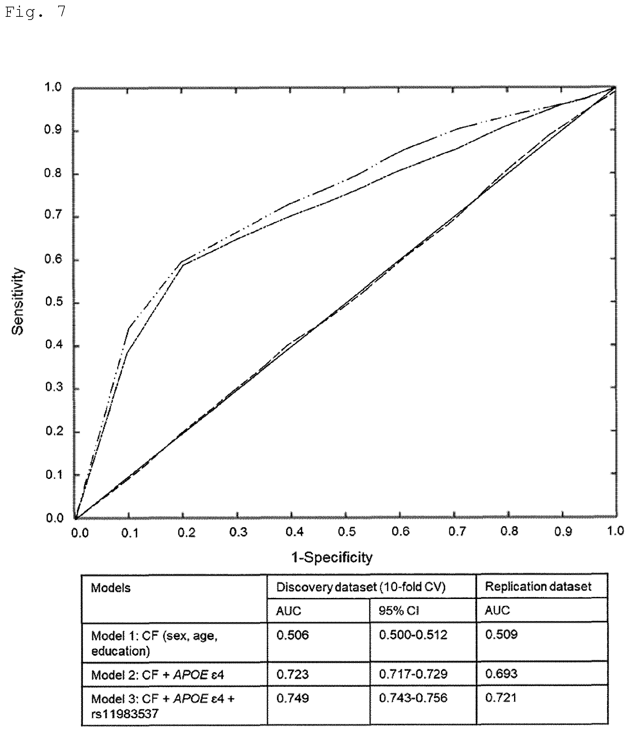

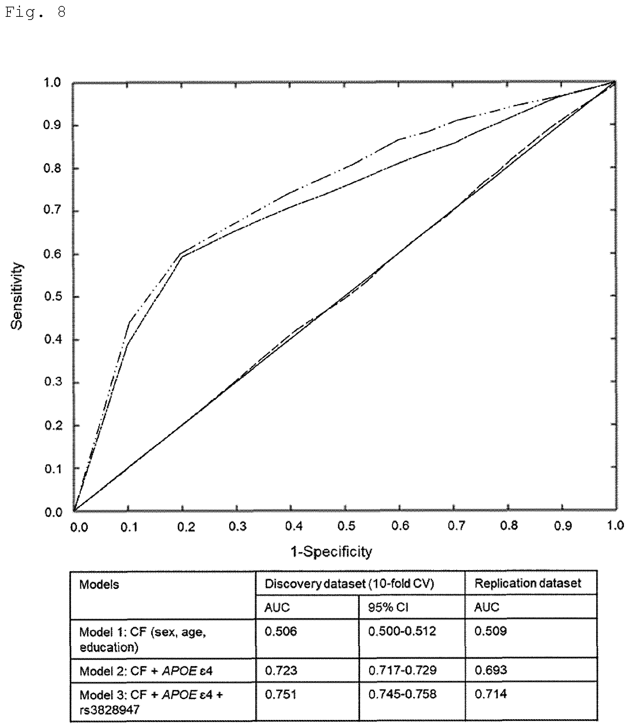

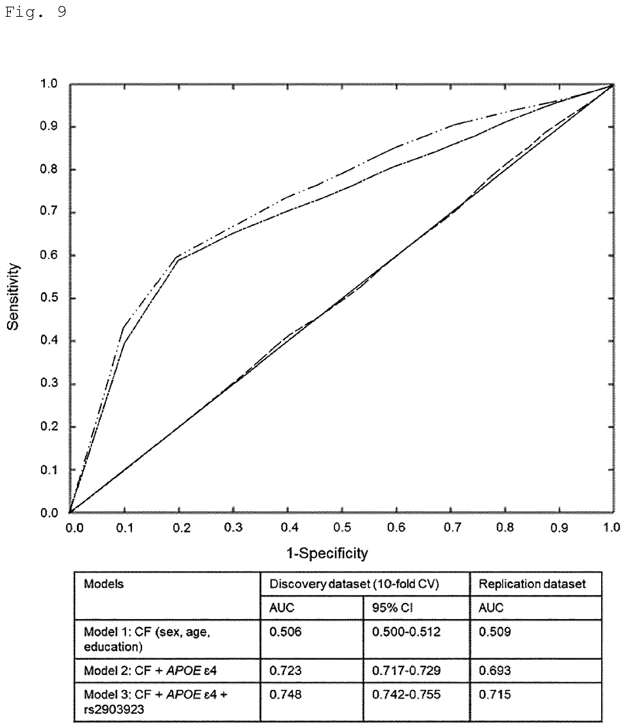

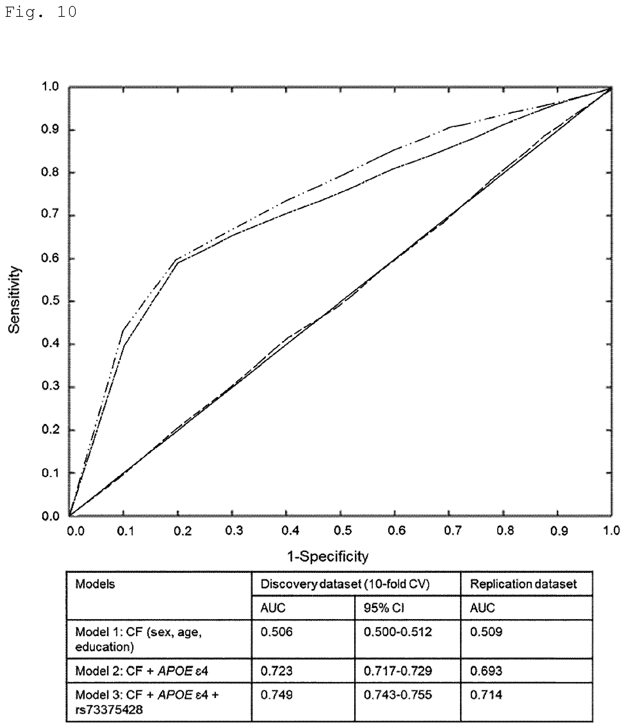

Several prediction models were developed to test the clinical utility of the APOE ε4 allele, and each model for the six SNPs (rs6978259, rs6958464, rs11983537, rs3828947, rs2903923 and rs73375428) was developed (see Equation 1). Referring to FIG. 5, in the 10-fold cross-validation with 100 repetitions, model 1 including only individual characteristics (age, sex, and level of education) showed an AUC of 0.506 (95% CI = 0.500-0.512) . Model 2 obtained by incorporating the APOE ε4 allele in model 1 showed increased prediction performance (AUC = 0.723; 95% CI = 0.717-0.729), and model 3 (Equation 1) obtained by incorporating rs6978259 in model 2 showed further increased prediction performance (AUC = 0.748; 95% CI = 0.742-0.755). When each model, trained in the data for model development, was tested in the data for validation, model 3 including individual characteristics, APOE ε4 genotype and rs6978259 showed a higher AUC value than model 1 and model 2 (model 1 AUC = 0.509, model 2 AUC = 0.693, and model 3 AUC = 0.713). Referring to FIGS. 6 to 10, similar results were also observed for rs6958464, rs11983537, rs3828947, rs2903923 and rs73375428.

### ③ Analysis of function

Finally, the present inventors characterized the function of the six identified SNPs by leveraging bioinformatic tools and previously reported results. First, the present inventors checked whether MAF of SNPs identified in this Example was similar to the MAF in the East Asian population using the 1000 Genomes Project dataset. To evaluate the genotype-specific expression of the identified SNPs in human brain tissues, the present inventors performed cis-expression quantitative trait loci (cis-eQTL) analysis through the Genotype-Tissue Expression portal (https:// gtexportal.org) .

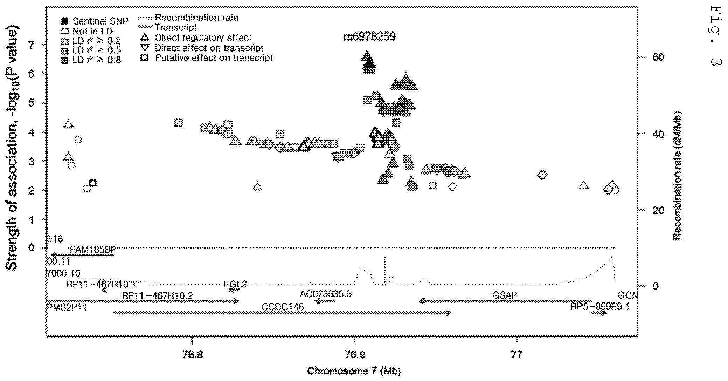

As a result, as shown in FIG. 3, rs6978259 was found to be located in the intron of the coiled-coil domain containing 146 (CCDC146) gene.

These results suggest that there are differences in the types of SNPs that are highly associated with brain amyloid-beta deposition due to differences in cohorts, and that APOE ε4 genotype as a blood biomarker and at least one SNP genotype selected from the group consisting of rs73375428, rs6978259, rs2903923, rs3828947, rs6958464 and rs11983537 are combined and analyzed with individual characteristics including sex, age, and education level for Koreans, it is possible to effectively screen individuals with a high likelihood of brain amyloid-beta deposition at an early stage.

So far, the present disclosure has been described with reference to the embodiments. Those of ordinary skill in the art to which the present disclosure pertains will appreciate that the present disclosure may be embodied in modified forms without departing from the essential characteristics of the present disclosure. Therefore, the disclosed embodiments should be considered from an illustrative point of view, not from a restrictive point of view. The scope of the present disclosure is defined by the claims rather than the foregoing description, and all differences within the scope equivalent thereto should be construed as being included in the present disclosure.

### Claims

1. A method of providing information for predicting likelihood of brain amyloid-beta deposition, the method comprising steps of:

    a) collecting individual characteristics including sex, age, and education level from an individual;
    b) measuring an APOE ε4 genotype and at least one SNP genotype selected from the group consisting of rs73375428, rs6978259, rs2903923, rs3828947, rs6958464, and rs11983537, in a biological sample from the individual; and
    c) predicting the likelihood of brain amyloid-beta deposition by combining the collected individual characteristics and the measured APOE ε4 genotype and SNP genotype.

2. The method of claim 1, wherein the biological sample in step b) is one selected from the group consisting of whole blood, plasma, saliva, oral mucosa, and hair.

3. The method of claim 1, wherein step c) comprises setting the individual characteristics, the APOE ε4 genotype and the SNP genotype as independent variables, setting specific coefficients for them as dependent variables, and preparing a brain amyloid-beta deposition prediction model through statistical analysis.

4. The method of claim 3, wherein the statistical analysis is performed using an analysis method selected from the group consisting of linear or nonlinear regression analysis method, linear or nonlinear classification analysis method, ANOVA, neural network analysis method, deep neural network analysis method, genetic analysis method, support vector machine analysis method, hierarchical analysis or clustering analysis method, hierarchical algorithm or Kernel principal components analysis method using a decision tree, Markov Blanket analysis method, recursive feature elimination or entropy-based recursive feature elimination analysis method, forward floating search or backward floating search analysis method, and combinations thereof.

5. The method of claim 3, wherein the brain amyloid-beta deposition prediction model is represented by Equation 1 below.

```
[Equation 1]

Aβ deposition = -0.90 + (0.0026*age) + (0.164*sex) +

(0.0177*education level) + (1.653*APOE ε4 genotype) + (α*SNP

genotype)
```

wherein α is -0.612 when SNP is rs6978259, -0.652 when SNP is rs6958464, -0.658 when SNP is rs11983537, -0.571 when SNP is rs3828947, -0.612 when SNP is rs2903923, and -0.633 when SNPP is rs73375428.

6. A composition for predicting likelihood of brain amyloid-beta deposition, the composition containing an agent capable of detecting an APOE ε4 genotype and at least one SNP genotype selected from the group consisting of rs73375428, rs6978259, rs2903923, rs3828947, rs6958464, and rs11983537.

7. The composition of claim 6, wherein the agent is at least one selected from the group consisting of a primer set, a probe, and an antisense oligonucleotide, which specifically bind to a nucleotide sequence comprising the APOE ε4 genotype or the SNP genotype.

8. A kit for predicting likelihood of brain amyloid-beta deposition, the kit comprising the composition of claim 6.

Fig. 1

Fig. 2

Fig. 3

Fig. 4

Fig. 5

| Models | Discovery dataset (10-fold CV) | | Replication dataset |
|---|---|---|---|
| | AUC | 95% CI | AUC |
| Model 1: CF (sex, age, education) | 0.506 | 0.500-0.512 | 0.509 |
| Model 2: CF + *APOE* ε4 | 0.723 | 0.717-0.729 | 0.693 |
| Model 3: CF + *APOE* ε4 + rs6978259 | 0.748 | 0.742-0.755 | 0.713 |

Fig. 6

| Models | Discovery dataset (10-fold CV) | | Replication dataset |
|---|---|---|---|
| | AUC | 95% CI | AUC |
| Model 1: CF (sex, age, education) | 0.506 | 0.500-0.512 | 0.509 |
| Model 2: CF + *APOE* ε4 | 0.723 | 0.717-0.729 | 0.693 |
| Model 3: CF + *APOE* ε4 + rs6958464 | 0.749 | 0.742-0.755 | 0.713 |

Fig. 7

| Models | Discovery dataset (10-fold CV) | | Replication dataset |
|---|---|---|---|
| | AUC | 95% CI | AUC |
| Model 1: CF (sex, age, education) | 0.506 | 0.500-0.512 | 0.509 |
| Model 2: CF + *APOE ε4* | 0.723 | 0.717-0.729 | 0.693 |
| Model 3: CF + *APOE ε4* + rs11983537 | 0.749 | 0.743-0.756 | 0.721 |

Fig. 8

| Models | Discovery dataset (10-fold CV) | | Replication dataset |
|---|---|---|---|
| | AUC | 95% CI | AUC |
| Model 1: CF (sex, age, education) | 0.506 | 0.500-0.512 | 0.509 |
| Model 2: CF + *APOE* ε4 | 0.723 | 0.717-0.729 | 0.693 |
| Model 3: CF + *APOE* ε4 + rs3828947 | 0.751 | 0.745-0.758 | 0.714 |

Fig. 9

| Models | Discovery dataset (10-fold CV) | | Replication dataset |
|---|---|---|---|
| | AUC | 95% CI | AUC |
| Model 1: CF (sex, age, education) | 0.506 | 0.500-0.512 | 0.509 |
| Model 2: CF + *APOE* ε4 | 0.723 | 0.717-0.729 | 0.693 |
| Model 3: CF + *APOE* ε4 + rs2903923 | 0.748 | 0.742-0.755 | 0.715 |

Fig. 10

| Models | Discovery dataset (10-fold CV) | | Replication dataset |
|---|---|---|---|
| | AUC | 95% CI | AUC |
| Model 1: CF (sex, age, education) | 0.506 | 0.500-0.512 | 0.509 |
| Model 2: CF + *APOE* ε4 | 0.723 | 0.717-0.729 | 0.693 |
| Model 3: CF + *APOE* ε4 + rs73375428 | 0.749 | 0.743-0.755 | 0.714 |

# INTERNATIONAL SEARCH REPORT

| International application No. |
|---|
| **PCT/KR2021/015884** |

| | |
|---|---|
| **A.** | **CLASSIFICATION OF SUBJECT MATTER** |

**G16B 40/10**(2019.01)i; **G16B 5/00**(2019.01)i; **G16B 20/20**(2019.01)i; **G16B 30/10**(2019.01)i; **G16H 50/50**(2018.01)i; **G16H 50/70**(2018.01)i

According to International Patent Classification (IPC) or to both national classification and IPC

| | |
|---|---|
| **B.** | **FIELDS SEARCHED** |

Minimum documentation searched (classification system followed by classification symbols)

G16B 40/10(2019.01); A61K 31/4412(2006.01); C07K 17/00(2006.01); C12Q 1/68(2006.01); G01N 24/08(2006.01); G01N 33/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: APOE ε4, rs73375428, rs6978259, rs2903923, rs3828947, rs6958464, rs11983537, 단일 염기 다형성(single nucleotide polymorphism, SNP), 유전자형(genotype), 아밀로이드 베타(amyloid beta), 축적(deposition), 뇌(brain)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| A | BAEK, Min Seok et al. Effect of APOE ε4 genotype on amyloid-β and tau accumulation in Alzheimer's disease. Alzheimer's Research & Therapy. 31 October 2020 (online publication date), vol. 12, article no. 140, inner pp. 1-12.<br>See inner pages 1-10. | 1-8 |
| A | WO 2018-148788 A1 (CRC FOR MENTAL HEALTH LTD) 23 August 2018 (2018-08-23)<br>See abstract; and claims 1-27. | 1-8 |
| A | LIM, Yen Ying et al. APOE genotype and early β-amyloid accumulation in older adults without dementia. Neurology. 09 August 2017 (online publication date), vol. 89, article no. 10, inner pp. 1-7.<br>See inner pages 1-6. | 1-8 |
| A | KR 10-0279898 B1 (DUKE UNIVERSITY) 02 April 2001 (2001-04-02)<br>See abstract; and claims 1-41. | 1-8 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **18 February 2022** | **21 February 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2021/015884**

### C. DOCUMENTS CONSIDERED TO BE RELEVANT

| Category* | Citation of document, with indication, where appropriate, of the relevant passages | Relevant to claim No. |
|---|---|---|
| A | KR 10-2020-0047371 A (SEOUL NATIONAL UNIVERSITY R&DB FOUNDATION) 07 May 2020 (2020-05-07)<br>    See abstract; and claims 1-10. | 1-8 |
| PX | KIM, Hang-Rai et al. Identifying novel genetic variants for brain amyloid deposition: a genome-wide association study in the korean population. Alzheimer`s Research & Therapy. 21 June 2021 (online publication date), vol. 13, article no. 117, inner pp. 1-11.<br>    See inner pages 1-9. | 1-8 |

## INTERNATIONAL SEARCH REPORT
### Information on patent family members

International application No.

**PCT/KR2021/015884**

| Patent document cited in search report | | | Publication date (day/month/year) | Patent family member(s) | | | Publication date (day/month/year) |
|---|---|---|---|---|---|---|---|
| WO | 2018-148788 | A1 | 23 August 2018 | None | | | |
| KR | 10-0279898 | B1 | 02 April 2001 | AT | 262592 | T | 15 April 2004 |
| | | | | AU | 5350094 | A | 09 May 1994 |
| | | | | AU | 677614 | B2 | 01 May 1997 |
| | | | | CA | 2142300 | A1 | 28 April 1994 |
| | | | | CA | 2142300 | C | 23 August 2005 |
| | | | | CN | 1092525 | A | 21 September 1994 |
| | | | | CN | 1525168 | A | 01 September 2004 |
| | | | | DE | 625212 | T1 | 02 November 2000 |
| | | | | DE | 69333461 | T2 | 24 March 2005 |
| | | | | EP | 0625212 | A1 | 23 November 1994 |
| | | | | EP | 0625212 | B1 | 24 March 2004 |
| | | | | ES | 2148116 | T1 | 16 October 2000 |
| | | | | FI | 951701 | A0 | 10 April 1995 |
| | | | | FI | 951701 | D0 | 10 April 1995 |
| | | | | JP | 07-502418 | A | 16 March 1995 |
| | | | | JP | 3265577 | B2 | 11 March 2002 |
| | | | | NO | 951383 | A | 07 April 1995 |
| | | | | NO | 951383 | D0 | 07 April 1995 |
| | | | | NO | 951383 | L | 07 April 1995 |
| | | | | NZ | 257215 | A | 20 December 1996 |
| | | | | US | 05508167 | A | 16 April 1996 |
| | | | | US | 05716828 | A | 10 February 1998 |
| | | | | US | 05767248 | A | 16 June 1998 |
| | | | | US | 06027896 | A | 22 February 2000 |
| | | | | WO | 94-09155 | A1 | 28 April 1994 |
| | | | | ZA | 9307536 | A | 03 May 1994 |
| | | | | ZA | 937536 | B | 03 May 1994 |
| KR | 10-2020-0047371 | A | 07 May 2020 | EP | 3872494 | A1 | 01 September 2021 |
| | | | | KR | 10-2254053 | B1 | 20 May 2021 |
| | | | | WO | 2020-085803 | A1 | 30 April 2020 |

Form PCT/ISA/210 (patent family annex) (July 2019)

**REFERENCES CITED IN THE DESCRIPTION**

**Patent documents cited in the description**

- KR 102130929
- KR 101989695

- KR 1020200073156

**Non-patent literature cited in the description**

- **YAN Q et al.** *Mol Psychiatry.,* 2018, 1-13
- **APOSTOLOVAL LG et al.** *JAMA Neurol.,* 2018, 328-341

- **RAGHAVAN NS et al.** *JAMA Neurol.,* 2020, 1288-1298